# EUROPEAN PATENT SPECIFICATION

(11) **EP 2 034 991 B1**
(45) Date of publication and mention of the grant of the patent: **15.02.2012**
(21) Application number: 07785895.9
(22) Date of filing: 04.07.2007
(51) Int. Cl.: A61K 31/4045, A61K 31/353, A61K 36/48, A61P 15/12

(54) **PREPARATION FOR PREVENTING AGEING SYMPTOMS CONTAINING PHYTOESTROGENS AND MELATONIN**
ZUBEREITUNG ZUR VERHINDERUNG DER ALTERUNGSSYMPTOME MIT PHYTOÖSTROGENEN UND MELATONIN
PRÉPARATION EMPÊCHANT L'APPARITION DES SYMPTOMES DU VIEILLISSEMENT

(30) Priority: 04.07.2006 EP 06425463
(43) Date of publication of application: 18.03.2009
(73) Proprietor: I.R.Med. S.r.l., 37127 Avesa (Verona) (IT)
(72) Inventor: CAVAZZANA, Leonardo, 37124 Verona (IT)
(74) Representative: Ferreccio, Rinaldo
(86) International application number: PCT/EP2007/005917
(87) International publication number: WO 2008/003475

(56) References cited:
- WO-A-98/21947
- SECRETO GIORGIO ET AL: "Soy isoflavones and melatonin for the relief of climacteric symptoms: A multicenter, double-blind, randomized study." MATURITAS, vol. 47, no. 1, 20 January 2004 (2004-01-20), pages 11-20, XP002409483 ISSN: 0378-5122
- SETCHELL KENNETH D R ET AL: "Pharmacokinetics of a slow-release formulation of soybean isoflavones in healthy postmenopausal women" JOURNAL OF AGRICULTURAL AND FOOD CHEMISTRY, vol. 53, no. 6, March 2005 (2005-03), pages 1938-1944, XP002409484 ISSN: 0021-8561
- LANE E A ET AL: "Pharmacokinetics of melatonin in man: first pass hepatic metabolism" JOURNAL OF CLINICAL ENDOCRINOLOGY AND METABOLISM, ENDOCRINE SOCIETY, CHEVY CHASE, MD, US, vol. 61, no. 6, 1985, pages 1214-1216, XP002990677 ISSN: 0021-972X
- BELLIPANNI F ET AL.: "Effects of Melatonin...", ANN N Y ACAD SCI, vol. 1057, 2005, pages 393-402,
- BELLIPANNI F ET AL.: "Effects of melatonin...", EXP GERONTOLOGY, vol. 36, 2001, pages 297-310,

## Description

### Field of application

The present invention regards a preparation for reducing the symptoms of menopause, such as for example hot flashes and night sweating.

### Prior art

The effects of ageing for the human body and in particular for the female body are known.

The ageing of the female body occurs in parallel with the definitive end of the reproductive capacity, i.e. menopause. In other words, a general ageing of the tissues in a female body occurs at the same time as an end of the reproductive capacity.

From the hormonal standpoint, there is in this phase a progressive incapacity to produce estrogens, and at the same time a progressive slowing of the activity of other hormones which regulate the activity of the productive system, above all GnRH, produced by the hypothalamus, whose secretion peaks result reduced in amplitude and frequency.

This lack of hormonal activity causes more or less intense discomfort in the female body, such as hot flashes, night sweating, tingling and nervous disturbances (irritability, sudden mood swings etc.) which can be very troublesome and often difficult to handle during everyday life.

It has also been noted by many epidemiological studies that Asian women are less hit by such disturbances (18% versus 70-80% of European women) and that a diet particularly rich in phytoestrogens is responsible for these beneficial consequences.

The phytoestrogens are compounds of vegetable origin, present in about 300 plants. By definition they are molecules of vegetable origin provided with estrogenic action, even if much less powerful - in the range of one-thousandth to one-ten-thousandth (1/1,000 - 1/10,000) - than that of estradiol, the main female estrogen hormone.

In fact, even if with smaller affinity, the phytoestrogens, when taken with food, bind to the estrogen receptor in the body, forming a receptorial complex which functions in a similar manner.

For example, the main class of phytoestrogens is represented by the phenolic phytoestrogens, whose main skeleton is referable to the diphenylpropane, and among the phenolic phytoestrogens the isoflavones are those provided with a higher estrogenic activity with respect to the other phytoestrogens. Very rich sources of isoflavones ar soy seeds.

Many of the healthy properties of the isoflavons are ascribable to the estrogenic action of genistein and daidzein, the two most important isoflavones, and to their interaction with the estrogen receptors distributed throughout the body. In particular, genistein is considered to be the most active isoflavone with regard to estrogenic activity.

For these reasons, the phytoestrogens are today considered a non-negligible complementary source of estrogen for preventing the menopause symptoms, and in particular the administration of a soy extract, which is rich in isoflavones capable of an action similar to estrogen, is able to reduce the symptoms of menopause, both in terms of intensity and frequency.

For the administration, preparations for food supplements including phytoestrogens are currently on the market, such as for example isoflavones extracted from soy flour or from soy flakes. The known preparations, while advantageous in many aspects, nevertheless have several drawbacks which are still not resolved.

The main drawback lies in the fact that the known products are not always effective in reducing all of the menopause symptoms in a constant manner over the entire span of the day, i.e. 24 hour cycle. For example, the phytoestrogens by themselves are not effective in preventing the hot flashes, which maintain a considerable intensity in the late afternoon and during the night.

A composition has also been proposed (patent application WO 98/21947) comprising a mixture of isoflavones and melatonin, which nevertheless was not effective in overcoming the aforesaid drawback.

Secreto G. et al "Soy isoflavones and melatonin for the relief of climacteric symptoms: a multicenter, double-blind, randomized study" MATURITAS, Vol. 47, No. 1, 20.01.2004, pp. 11-20, disclose the evaluation of the effect of soy isoflavones and melatonin in relieving menopausal symptoms.

In this study, the patients of one out of four groups (Group 1) were asked to take for 90 days at midday a capsule containing 40 mg isoflavones and in the evening another capsule containing 40 mg isoflavones and 3 mg melatonin. The isoflavones used in this study contained 50 % diadzein, 35% glycitein and 15% genistein.

The patients of other two groups took, both at midday and in the evening, one capsule containing 40 mg isoflavones (Group 2) and placebo (Group 4), respectively.

The patients of Group 3 took one capsule of placebo at midday and one capsule of melatonin (3 mg) in the evening.

The authors of this study concluded that they did not find any appreciable improvements in menopausal symptoms from intake of soy isoflavones and melatonin over placebo.

Bellipanni et al., "Effects of melatonin in perimenopausal and menopausal women", Ann. N. Y. Acad. Sci. 1057: 393-402 (2005) and Bellipanni et al., "Effects of melatonin in perimenopausal and menopausal women: a randomized and placebo controlled study", Experimental Gerontology 36 (2001), 297-310, pp. 297-310, disclose that a six-month treatment with 3 mg melatonin, taken once a day at bedtime, produced an improvement of thyroid function, positive changes of gonadotropins and abrogation of menopause-related depression.

The technical problem underlying the present invention therefore lies in making available a preparation comprising phytoestrogens capable of preventing, in an effective manner, the menopausal symptoms, in particular the hot flashes, during the entire daily cycle.

### Summary of the invention

The solution idea underlying the present invention lies in the identification of a correlation between the menopause symptoms, and still more in particular hot flashes, and the reduction, caused by ageing, of the capacity of an individual to maintain an effective rhythmicity of the physiological functions in the 24 hour span, or circadian rhythm, i.e. the set of periodic manifestations which are verified with an event occurrence of about every twenty-four hours.

Based on this solution idea, the aforesaid technical problem is resolved by a preparation according to claim 1.

The soy isoflavones of the preparation according to the invention have generally a genistein content of 50-60%, typically about 50%.

A typical soy isoflavones composition useful for the purposes of the present invention includes 50% genistein, 35% diadzein and 15% glycitein and is provided by the product Novasoy powder, produced by ADM (Illinois), which contains 40% isoflavones .

In substance, the preparation is a product which according to the invention comprises two structurally distinct dosage units, i.e. single doses, for supplementing the body on one hand with phytoestrogens, which as said confront the hormonal problem, and on the other hand with melatonin, which confronts the alterations of the circadian cycle.

It is in fact known that the melatonin, which is *per se* naturally produced in the body by the pineal gland, is considered necessary for an effective circadian organisation. When, with ageing, there is a diminution of its hematic concentration, a food supplement of melatonin is necessary so to not to jeopardise the circadian cycle itself. According to one aspect of the invention, the use of two distinct dosage units offers the advantage that the two units can be administered according to a predetermined timing or rhythm which is a function of the biological time and rhythm that each compound (estrogen and melatonin) has in the body for carrying out its own action.

The reasons are the following.

Melatonin and phytoestrogens are *per se* different compounds, i.e. having a different activation rhythm in the body for reaching a specific hematic level or concentration.

It is known in fact that the secretion of the melatonin by the pineal gland is not uniform during the 24 hours, but higher secretions are verified in the hours of darkness, and in a particular manner the hours of the night, while the hematic concentration of the melatonin is practically insignificant during the daylight hours. For this reason, the melatonin must be administered with the arrival of darkness, therefore preferably in the evening, so to keep its level high when it is dark. After the administration, an increase of the hematic melatonin level is encountered, in a relatively short time, after about 1-2 hours from the administration (see "Melatonina la pillola della giovinezza" ["Melatonin the pill of youth"] by Maurizio Nordio ed. Aporie, "Melatonina, Fatti e fantasie" ["Melatonin, Facts and fantasies"]; Author: Tarquini Brunetto; Editor: Idelson-Gnocchi; Date of Publication: 1996"].

On the other hand, the activity of the phytoestrogens in general and thus also that of the isoflavones, i.e. attaining a high concentration in the blood, grows gradually after the administration with relatively longer times with respect to the melatonin until it reaches a maximum concentration peak. A. Cassidy "Physiological effects of phytoestrogens in relation to cancer and other human health risks" describes the metabolism of the phytoestrogens, and demonstrates the presence of phytoestrogens in the plasma and in urine after administration.

Still more in particular, the presence in the blood of the orally-administered phenolic phytoestrogens was widely demonstrated, both for the compounds and for their metabolites. The phenolic phytoestrogens are present in the blood both in free form and conjugated with glucuronic acid; a portion of these is secreted in the bile and enters into the enterohepatic circulation exactly as do the steroid estrogens, and still like the latter the urine is the main elimination vehicle with a half-life of less than 24 hours (Barns S, Sfakianos J, Coward L, Kirk M., "Soy isoflavonoids and cancer prevention. Underlying biochemical and pharmacological issues", Adv Experiment Med Biol. 1996; 401:87-100 Braden AWH Hart NK, Lamberton JA, "The estrogenic activity of and metabolism of certain isoflavones in sheep", Austral J Agricult res 1967;18:355-8, Hackett, AM, "The metabolism of flavonoid compounds in mammals", In: Liss, AR, ed. Plant Flavonoids in Biology and Medicine: Biochemical, Pharmacological, and Structural-Activity Relationships New York; Alan R. Liss, 1986, 177-96; Nilsson A, "Demethylation of the plant oestrogen biochanin A in the rat", Nature 1961; 192:358. Kelly GE, Nelson C, Waring MA, et al, "Metabolites of dietary (soya) isoflavones in human urine", Clinica Chimica Acta 1993; 223:9-22; Joannou GE, Kelly GE, Reeder AY, et al, "A urinary profile study of dietary phytoestrogens. The identification and mode of metabolism of new isoflavonoids", J Steroid Biochem Molec Biol 1995; 54:167-84.).

It was therefore found that the phytoestrogens in general take more time to reach a maximum concentration, or bioavailability, in the blood than the melatonin.

For this reason, in order to obtain a maximum synergy between the two components, the two dosage units are separately administered, and in particular the soy isoflavones of the first dosage unit are first administered in a moment of the day which is chosen such that when the melatonin is administered (in dark conditions or in the evening), the soy isoflavones are already present in the blood with a predetermined concentration.

In such a manner, the melatonin is administered on one hand at the moment in which the body requires it (darkness), and on the other hand finds the isoflavones of the first dosage unit already present in the blood, without interfering with their estrogenic action. In other words, the melatonin is administered when the concentration of isoflavones in the blood is high, and when the melatonin request in the body is high.

Due to the two distinct dosage units, the administration according to a predetermined regimen at different times is thus permitted, as is the achievement of an effective rhythmicity of the estrogenic function of the isoflavones in the body.

Moreover, since after the peak the concentration of the isoflavones progressively decreases, in order to compensate this diminution of concentration and to maintain the constant level, the second dosage unit also comprises soy isoflavones in addition to the melatonin. In other words, the second dosage unit comprises a mixture of melatonin and soy isoflavones, such that the natural diminution of the high hematic level of isoflavones achieved with the administration of the first dosage unit is compensated by the progressive passage in circulation of the isoflavones taken together with the melatonin in the second dosage unit, with the net result of a maintenance of this high hematic level.

The isoflavone amount of the first and second dosage unit is preferably in the range of 30-200 mg, more preferably between 30-100 mg, still more preferably 40 mg.

The melatonin amount of the second dosage unit is preferably 5 mg.

The dosage units are pharmaceutical forms for oral intake of gastro-resistant type, i.e. capable of maintaining themselves intact during the residence in the stomach and allowing a breakdown in the intestinal tract. To such end the dosage units are composed of capsules in soft gelatin, or pearls, made according to pharmaceutical technique. It is clear that within the scope of the present invention, any one of the known gastro-resistant pharmaceutical dosage forms can be used, available to a man skilled in the art of pharmaceutical preparations.

It should be noted that the isoflavones of soy have a concentration peak 12 hours after intake, and subsequently such concentration decreases to a minimum 24 hours after intake, with a minimum presence of isoflavones also 48-72 hours after intake. Keeping in mind this concentration rhythm, the first dosage unit containing soy isoflavones is taken in the morning, for example at 8 am, and the second dosage unit comprising a mixture of melatonin and isoflavones is subsequently taken at least 8 hours after, at a time in which the body requires a melatonin supplement, for example with the arrival of darkness, around sunset, or when melatonin variations in the bodies have been verified, induced by sudden time zone changes.

Preferably, the intake of the second dosage unit occurs 12 hours later, i.e. at the maximum concentration of isoflavones in the blood.

The isoflavones of the second dosage unit in this manner compensate the diminution of isoflavones of the first dosage unit. With the two dosage units, the entire 24 hour span is therefore covered, with the presence of the isoflavones maintained constantly high and the insertion of the melatonin activity at the moment of maximum isoflavone peak, and at the same time ensuring a high hematic level of melatonin in the night hours.

In particular, in a preferred solution, the preparation comprises a first dosage unit including 40 mg of soy isoflavones, while the second dosage unit includes 40 mg of soy isoflavones and 5 mg of melatonin. Each dosage unit is a capsule in soft gelatin capable of overcoming the gastric barrier. Each capsule contains in addition to the active components a vitamin complex including vitamins of the B group, such as Vitamin B1, B2 and B6, together with Vitamin C and E.

The soft gelatin capsules moreover comprise acceptable pharmaceutical carriers, media and additives of various known type, for example vegetable oils, such as non-genetically modified soy oil, soy lecithin, resistance and thickener agents, metered in accordance to the prescriptions of the pharmacopoeia.

In accordance with this preferred solution, it should be remarked that, as stated above, to cover the 24 hours of phytoestrogen activity, the intake of only the two (first and second) dosage units is sufficient, without further dosage units, and the programmed intake is repeated over the following days. For this reason, the preparation contains a plurality of first and second dosage units in equal numbers, i.e. in a 1:1 ratio between the first dosage unit and the second dosage unit.

In order to ensure a correct intake of the two dosage units, and at the predetermined times, the preparation can be included in a package on which, on a visible surface, the intake modes of the two dosage units are indicated according to the abovementioned timing. Alternatively, the intake modes are indicated in an illustrative sheet.

The present invention moreover refers to a composition comprising melatonin for use in relieving menopausal symptoms according to claim 7.

Essentially, the administration of the two components (soy isoflavones and melatonin) is chosen based on the times and biological rhythms which such components have in the human body. The use according to the invention permits in fact respecting such biological times in the body, so to not alter its rhythms, but also maintain its correct mechanism.

Preferably, the first dosage unit is administered at the beginning of the day while the second dosage unit is administered with the arrival of darkness, preferably in the evening.

It has been experimentally found that the preparation according to the invention, contrary to that used in the aforementioned study by Secreto et al, is effective in relieving the menopausal symptoms.

This is likely to be due to the different composition of the employed soy isoflavones, which in the present invention include at least 50% genistein, whereas the soy isoflavones used by Secreto et al only contain 15% genistein, which is the isoflavone with the most prominent estrogen-like activity. The insufficient amount of melatonin used by Secreto et al (3 mg) can also be an important factor.

Further characteristics and advantages of the preparation according to the invention will be clear from the following description of an embodiment thereof, given as indicative and not limiting with reference to the attached drawing.

### Brief description of the figures

Figure 1 illustrates the rate of the hematic concentration of the isoflavones and melatonin of the single dosage units in the body.
Figure 2 illustrates the rate of the hematic concentration of the isoflavones and melatonin, wherein the hematic concentration of the isoflavones results from the summation of the isoflavones of the two dosage units.

### EMBODIMENT EXAMPLE

A first soft gelatin capsule (first dosage unit), also called pearl, is prepared in the following manner.

40 mg of isoflavones in powder extracted from soy flakes and available on the market are mixed with a vitamin complex, including vitamins of the B group, such as B1 (thiamine mononitrate: 1.2 mg), B2 (riboflavin: 1.3 mg) and B6 (pyridoxine hydrochloride: 2 mg), and other vitamins, such as Vitamin C (in powder: 60 mg) and Vitamin E (D-L-alpha-tocopherol: 15.0 mg).

Before mixing, the isoflavones are subjected to traditional treatments of the pharmaceutical industry for preventing the product's self-fermentation, in order to ensure the estrogenic activity, to prevent a possible bacterial attack and, essentially, to keep the soy extract intact.

To such end it is possible to use 100 mg of 40% Novasoy powder produced by ADM (Illinois) and containing 40% isoflavones - which have already been subjected to the aforesaid treatments. The composition of the isoflavones conatined in Novasoy powder is the following: genistein 50%, diadzein 35%, glycitein 15%.

In particular, in accordance with the correct pharmaceutical preparation technique, the isoflavones and the vitamin complex are mixed in non-genetically modified soy oil (345 mg) together with pharmaceutically acceptable media and additives, such as glycerylmonostearate (26 mg) and soy lecithin (18.8 mg) to form a pasty suspension.

The pasty suspension is subsequently encapsulated according to known techniques, for example the Scherer technique, or the Lambo technique, to form the soft gelatin capsule. In particular, the shell forming the capsule comprises gelatin (about 202 mg) and glycerin (about 64 mg).

A second capsule, or pearl, in soft gelatin (second dosage unit) is prepared in the following manner.

40 mg of isoflavones extracted from soy flakes, for example 100 mg of 40% Novasoy produced by ADM, which as mentioned above have been previously subjected to a conventional treatment for maintaining their properties integral, and which contain 40% isoflavones, are mixed with 5 mg of melatonin available on the market.

Isoflavones and melatonin are mixed with a vitamin complex, including vitamins of the B group, such as B1 (thiamine mononitrate: 1.2 mg), B2 (riboflavin: 1.3 mg) and B6 (pyridoxine hydrochloride: 2 mg), and other vitamins, such as Vitamin C (in powder: 60 mg) and Vitamin E (D-L-alpha-tocopherol: 15.0 mg).

In particular, in accordance with the correct pharmaceutical preparation technique, melatonin, the isoflavones and thevitamin complex are mixed in non-genetically modified soy oil (345 mg) together with pharmaceutically acceptable media and additives, such as glycerylmonostearate (26 mg) and soy lecithin (18.8 mg) to form a pasty suspension.

Also in this case, the pasty suspension is encapsulated according to known techniques, for example the Scherer technique, or the Lambo technique, to form the soft gelatin capsule. In particular, the shell forming the capsule comprises gelatin (about 202 mg) and glycerin (about 64 mg).

Subsequently, a plurality of first and second soft gelatin capsules are assembled in a single package. To distinguish the two dosage units, the soft gelatin capsules are coloured with different colours.

The package moreover bears instructions, which are printed on a surface of the package and regard the intake modes of the two dosage units over a 24-hour span.

In particular, the instructions indicate the intake of a first dosage unit at about 8 in the morning, and to take the second dosage unit at about 8 in the evening, i.e. 12 hours after the first dosage unit, or in any case one hour before going to sleep.

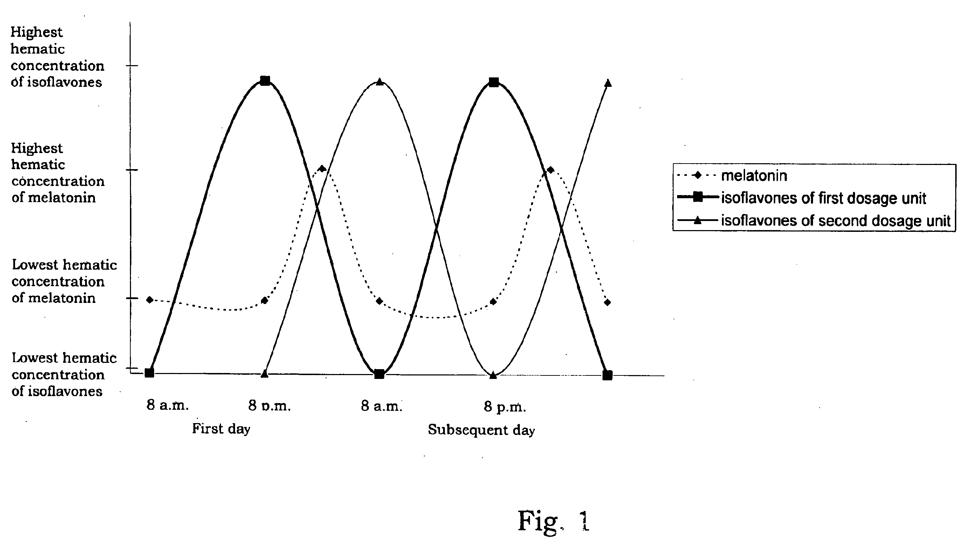

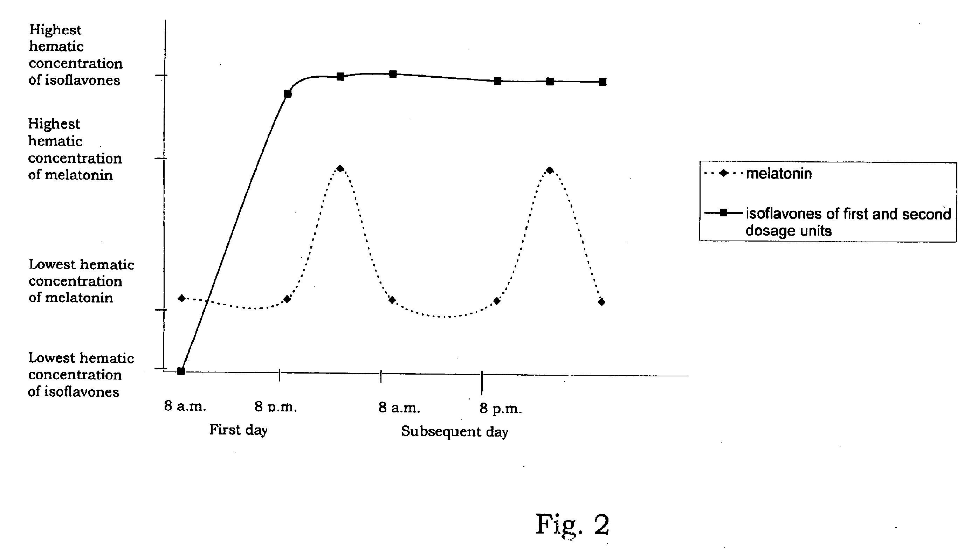

With reference to figure 1, it is possible to recognise, as schematic example, the course of the hematic concentration of the isoflavones and melatonin in the body after the intake at 8am (the first capsule) and 8pm (the second capsule), respectively, during a first and a subsequent day of treatment. The isoflavones of the first dosage unit have a hematic activity peak 12 hours after intake, at 8pm, when the second dosage unit is taken. After the 12 hours, the hematic level of the isoflavones of the first dosage unit decreases to a minimum at 24 hours after intake. The isoflavones of the second dosage unit have a hematic level progression which is of course equal to that of the first dosage unit, but staggered by 12 hours, and thus they have an increasing course for the first 12 hours, from 8pm to a hematic level peak at 8am, which compensates the decreasing course of the isoflavones of the first dosage unit. A substantially constant overall isoflavone level is thus obtained over the span of 24 hours (as illustrated in figure 2).

The melatonin of the second dosage unit (which is added to that naturally and cyclically produced by the body in the span of 24 hours) is inserted at the time of the maximum peak of the isoflavones in the blood of the first dosage unit, and thus exerts its action during the night, which then decreases towards the morning at 8am, such that a synergic and simultaneous action is performed by the two components (isoflavones on the one hand, and in particular genistein, and melatonin on the other) in the body to coincide with their maximum level peak.

For the subsequent days, continuing the programmed intake of the two capsules, the course of the isoflavone and melatonin concentrations described above are cyclically repeated.

The efficacy of the preparation according to the present invention has been evaluated in comparison with the preparation used in the aforementioned study by Secreto et al.

For this purpose, the following four batches of capsules were prepared:
a1) a batch of soft gelatine capsules (pearls), wherein each capsule contained 100 mg of 40% Novasoy powder (i.e. 40 mg soy isoflavones having the following composition: genistein 50%, diadzein 35%, glycitein 15%), 5 mg melatonin, and glycerol and soy lecithin as excipients;
a2) a batch of soft gelatine capsules (pearls), wherein each capsule contained 100 mg of 40% Novasoy powder (i.e. 40 mg soy isoflavones having the following composition: genistein 50%, diadzein 35%, glycitein 15%), and glycerol and soy lecithin as excipients;
b1) a batch of hard gelatine capsules, wherein each capsule contained 40 mg isoflavones (with the following composition: diadzein 50 %, glycitein 35% and genistein 15%), melatonin 3 mg, and sorbitol as the excipient.
b2) a batch of hard gelatine capsules, wherein each capsule contained 40 mg isoflavones (with the following composition: diadzein 50 %, glycitein 35% and genistein 15%), and sorbitol as the excipient.

50 postmenopausal women aged between 54 and 65 and showing menopausal symptoms voluntarily accepted to take part in the experimental trial described hereafter.

The patients were divided into two 25-women groups. The subjects of the first group (Group A) received a bottle a1 containing 90 soft capsules from batch a1) and a bottle a2 containing 90 capsules from batch a2), while the subjects of the second group (Group B) received a bottle b1 with 90 rigid capsules from batch b1) and a bottle b2 with 90 rigid capsules from batch b2)..

Each woman of group A was instructed to take one capsule from bottle a2 at 8.00 a.m. and one capsule from bottle a1 at 8.00 p.m. every day for 90 days.

Each woman of group B was instructed to take one capsule from bottle b2 at 8.00 a.m. and one capsule from bottle b1 at 8.00 p.m. every day for 90 days.

Before starting the treatment, the participants were requested to appraise the severity of a number of symptoms (somatic, vasomotor and psychological symptoms), with particular reference to nocturnal symptoms, such as insomnia and hot flashes, on suitable scales.

At the end of the treatment, the participants were asked to appraise again the same symptoms in the same way as at the time of starting the experiment, and to give an overall evaluation of the treatment.

A highly significant difference was noted for group A, between the scores given on the scale before starting the treatment and those given at the treatment end. The patients of group A also qualified the overall result of the treatment as "good" or "very good", in terms of relief of the menopausal symptoms.

On the contrary, no significant difference was noted for group B, between the scores given on the scale before starting the treatment and those given at the treatment end. The patients of group B also qualified the overall result of the treatment as "null" or "poor", in terms of relief of the menopausal symptoms.

Both groups A and B qualified the respective treatments as very good under the tolerability standpoint.

The above results confirm the importance of using soy isoflavones with an appropriate content of the most active isoflavone, that is genistein as well as the importance of using a sufficient amount of melatonin.

It is also confirmed that a treatment with the preparation according to the present invention allows a significant reduction of all menopause symptoms which are correlated with a decrease of the body's capacity to coordinate the rhythm of the biological functions, and in particular the circadian rhythm.

Of course, a man skilled in the art, in order to satisfy contingent and specific needs, may make numerous modifications and variants to the above described preparation and method, all moreover contained in the protective scope of the invention as defined by the following claims.

## Claims

1. Preparation for use in relieving menopausal symptoms in a human body, comprising a first dosage unit including soy isoflavones with a genistein content of at least 50% and a second dosage unit including melatonin and soy isoflavones with a genistein content of at least 50%, wherein the first and the second dosage units are different units to be separately administered from each other and wherein the melatonin amount of the second dosage unit is in the range of 5-10 mg, and wherein the first and the second dosage units further comprise vitamins B, C and E, and both dosage units are in the form of soft capsules or pearls of the gastro-resistant type.

2. Preparation for use according to claim 1, wherein the isoflavone amount of both dosage units is preferably in the range of 30-200 mg.

3. Preparation for use according to any one of the preceding claims, wherein the isoflavone amount of both units is 40 mg.

4. Preparation for use according to any one of the preceding claims, wherein the melatonin amount of the second dosage unit is 5 mg.

5. Preparation for use according to any one of the preceding claims, wherein the first dosage unit comprises at least 40 mg of soy isoflavones and the second dosage unit comprises at least 40 mg of soy isoflavones and 5 mg of melatonin.

6. Preparation for use according to any one of the preceding claims, wherein the two dosage units are in equal number, i.e. in a 1:1 ratio between the first dosage unit and the second dosage unit.

7. A composition comprising melatonin for use in relieving menopausal symptoms in a human body, wherein said composition comprises melatonin in an amount in the range of 5-10 mg and at least one soy isoflavone with a genistein content of at least 50%, and is administered according to a regimen which foresees the administration of said composition once a day with the appearance of darkness, at least 8 hours after isoflavones previously administered during the same day, and wherein said composition further comprises vitamins B, C and E, and is formulated in the form of soft capsules or pearls of the gastro-resistant type.

8. Composition for use according to claim 7, wherein the composition comprises 40 mg of soy isoflavones with a genistein content of at least 50% and about 5 mg of melatonin.

9. Preparation for use according to claims 1-6 wherein the first dosage unit and the second dosage unit are administered at different times as a function of the activity rhythm of the isoflavone and melatonin activity in the body.

10. Preparation for use according to claim 9, wherein the second unit is administered when darkness appears, and the first unit is administered at least 8 hours before the second dosage unit.

11. Preparation for use according to claim 10, wherein said first unit contains 40 mg of said soy isoflavones and said second unit contains 40 mg of said soy isoflavones and about 5 mg of melatonin.

12. Preparation for use according to any one of claims 9 to 11, wherein the first dosage unit is administered at the beginning of the day and the second dosage unit is administered in the evening about 12 hours after the first dosage unit.

## Patentansprüche

1. Präparat zur Anwendung zur Linderung von menopausalen Symptomen in einem menschlichen Körper, umfassend eine erste Dosierungseinheit, die Sojaisoflavone mit einem Genisteingehalt von mindestens 50% beinhaltet, und eine zweite Dosierungseinheit, die Melatonin und Sojaisoflavone mit einem Genisteingehalt von mindestens 50% beinhaltet, wobei die ersten und die zweiten Dosierungseinheiten verschiedene Einheiten sind, die voneinander getrennt verabreicht werden, und wobei die Melatoninmenge der zweiten Dosierungseinheit im Bereich von 5 bis 10 mg liegt und wobei die ersten und die zweiten Dosierungseinheiten außerdem Vitamine B, C und E umfassen und wobei beide Dosierungseinheiten in Form von Weichkapseln oder Perlen magensaftresistenter Art ausgebildet sind.

2. Präparat zur Anwendung nach Anspruch 1, wobei die Isoflavonmenge beider Dosierungseinheiten vorzugsweise im Bereich von 30 bis 200 mg liegt.

3. Präparat zur Anwendung nach einem der vorhergehenden Ansprüche, wobei die Isoflavonmenge beider Einheiten 40 mg beträgt.

4. Präparat zur Anwendung nach einem der vorhergehenden Ansprüche, wobei die Melatoninmenge der zweiten Dosierungseinheit 5 mg beträgt.

5. Präparat zur Anwendung nach einem der vorhergehenden Ansprüche, wobei die erste Dosierungseinheit mindestens 40 mg Sojaisoflavone umfasst und die zweite Dosierungseinheit mindestens 40 mg Sojaisoflavone und 5 mg Melatonin umfasst.

6. Präparat zur Anwendung nach einem der vorhergehenden Ansprüche, wobei die zwei Dosierungseinheiten in gleicher Anzahl vorliegen, das heißt in einem Verhältnis von 1:1 zwischen der ersten Dosierungseinheit und der zweiten Dosierungseinheit.

7. Zusammensetzung umfassend Melatonin zur Anwendung zur Linderung von menopausalen Symptomen in einem menschlichen Körper, wobei die Zusammensetzung Melatonin in einer Menge im Bereich von 5 bis 10 mg und mindestens ein Sojaisoflavon mit einem Genisteingehalt von mindesten 50% umfasst und nach einem Plan verabreicht wird, der die Verabreichung der Zusammensetzung einmal am Tag bei Einbruch der Dunkelheit vorsieht, mindestens 8 Stunden nachdem zuvor Isoflavone während desselben Tages verabreicht wurden, und wobei die Zusammensetzung außerdem Vitamine B, C und E umfasst und in Form von Weichkapseln oder Perlen magensaftresistenter Art abgefasst ist.

8. Zusammensetzung zur Anwendung nach Anspruch 7, wobei die Zusammensetzung 40 mg Isoflavone mit einem Genisteingehalt von mindestens 50 % und etwa 5 mg Melatonin umfasst.

9. Präparat zur Anwendung nach einem der Ansprüche 1 bis 6, wobei die erste Dosierungseinheit und die zweite Dosierungseinheit zu verschiedenen Zeiten als eine Funktion des Aktivitätsrhythmus der Isoflavon- und der Melatoninaktivität im Körper verabreicht werden.

10. Präparat zur Anwendung nach Anspruch 9, wobei die zweite Einheit bei Einbruch der Dunkelheit verabreicht wird und die erste Einheit mindestens 8 Stunden vor der zweiten Einheit verabreicht wird.

11. Präparat zu Anwendung nach Anspruch 10, wobei die erste Einheit 40 mg der Sojaisoflavone und die zweite Einheit 40 mg der Sojaisoflavone und etwa 5 mg Melatonin enthält.

12. Präparat zur Anwendung nach einem der Ansprüche 9 bis 11, wobei die erste Dosierungseinheit bei Tagesbeginn verabreicht wird und die zweite Dosierungseinheit am Abend etwa 12 Stunden nach der ersten Dosierungseinheit verabreicht wird.

## Revendications

1. Préparation destinée à être utilisée dans le soulagement des symptômes de la ménopause du corps humain, comprenant une première unité de dosage incluant des isoflavones de soja avec un contenu en génistéine d'au moins 50% et une deuxième unité de dosage incluant de la mélatonine et des isoflavones de soja avec un contenu en génistéine d'au moins 50%, **caractérisée en ce que** la première et la deuxième unités de dosage sont des unités différentes devant être administrées séparément l'une de l'autre et **en ce que** la quantité de mélatonine de la deuxième unité de dosage est comprise entre 5 et 10 mg, et **en ce que** la première et la deuxième unités de dosage comprennent en outre des vitamines B, C et E, et les deux unités de dosage sont sous la forme de capsules souples ou des perles de type gastro-résistantes.

2. Préparation pour une utilisation selon la revendication 1, **caractérisée en ce que** la quantité d'isoflavone des deux unités de dosage est de préférence comprise entre 30 et 200 mg.

3. Préparation pour une utilisation selon l'une quelconque des revendications précédentes, **caractérisée en ce que** la quantité d'isoflavone des deux unités est de 40 mg.

4. Préparation pour une utilisation selon l'une quelconque des revendications précédentes, **caractérisée en ce que** la quantité de mélatonine de la deuxième unité de dosage est de 5 mg.

5. Préparation pour une utilisation selon l'une quelconque des revendications précédentes, **caractérisée en ce que** la première unité de dosage comprend au moins 40 mg d'isoflavones de soja et la deuxième unité de dosage comprend au moins 40 mg d'isoflavones et 5 mg de mélatonine.

6. Préparation pour une utilisation selon l'une quelconque des revendications précédentes, **caractérisée en ce que** les deux unités de dosage sont en quantité égale, c'est-à-dire dans un ratio de 1 :1 entre la première unité de dosage et la deuxième unité de dosage.

7. Composition comprenant de la mélatonine destinée à être utilisée dans le soulagement des symptômes de la ménopause du corps humain, **caractérisée en ce que** ladite composition comprend de la mélatonine dans une quantité comprise entre 5 et 10 mg et au moins un isoflavone de soja avec un contenu en génistéine d'au moins 50% et est administrée selon un régime qui prévoit l'administration de ladite composition une fois par jour à la tombée de la nuit, au moins 8 heures après les isoflavones précédemment administrées dans la même journée et **caractérisée en ce que** la dite composition comprend en outre des vitamines B, C et E, et est formulée sous forme de capsules souples ou perles de type gastro-résistant.

8. Composition pour une utilisation selon la revendication 7, **caractérisée en ce que** la composition comprend 40 mg d'isoflavones de soja avec un contenu en génistéine d'au moins 50% et environ 5 mg de mélatonine.

9. Préparation pour une utilisation selon les revendications 1 à 6, **caractérisée en ce que** la première unité de dosage et la deuxième unité de dosage sont administrées à des moments différents en fonction du rythme d'activité de l'isoflavone et de la mélatonine dans le corps.

10. Préparation pour une utilisation selon la revendication 9, **caractérisée en ce que** la deuxième unité est administrée à la tombée de la nuit, et **en ce que** la première unité est administrée au moins 8 heures avant la deuxième unité de dosage.

11. Préparation pour une utilisation selon la revendication 10, **caractérisée en ce que** la dite première unité contient 40 mg desdits isoflavones de soja et **en ce que** ladite deuxième unité contient 40 mg desdits isoflavones de soja et environ 5 mg de mélatonine.

12. Préparation pour une utilisation selon l'une quelconque des revendications 9 à 11, **caractérisée en ce que** la première unité de dosage est administrée au début de la journée et **en ce que** la deuxième unité de dosage est administrée dans la soirée environ 12 heures après la première unité de dosage.
